# EUROPEAN PATENT SPECIFICATION

(11) **EP 2 403 894 B1**
(45) Date of publication and mention of the grant of the patent: **01.06.2016**
(21) Application number: 10749089.8
(22) Date of filing: 08.02.2010
(51) Int. Cl.: C08G 63/183, C08L 67/03, B65D 65/46, D01F 6/62, C08L 101/16

(54) **BIO-BASED POLYETHYLENE TEREPHTHALATE PACKAGING AND METHOD OF MAKING THEREOF**
POLYETHYLENTEREPHTHALATVERPACKUNG AUF BIOLOGISCHER BASIS UND HERSTELLUNGSVERFAHREN DAFÜR
EMBALLAGE EN POLY(TÉRÉPHTALATE D'ÉTHYLÈNE) D'ORIGINE BIOLOGIQUE ET SON PROCÉDÉ DE FABRICATION

(30) Priority: 12.10.2009 US 577480; 03.03.2009 WO PCT/US2009/035849
(43) Date of publication of application: 11.01.2012
(60) Divisional application: 16166900.7
(73) Proprietor: The Coca-Cola Company, Atlanta, GA 30313 (US)
(72) Inventor: KRIEGEL, Robert, M., Decatur GA 30030 (US); HUANG, Xiaoyan, Marietta GA 30068 (US); SCHULTHEIS, Mikell, Acworth GA 30102 (US); KOLLS, Brock, H, Alpharetta GA 30005 (US)
(74) Representative: Towler, Philip Dean
(86) International application number: PCT/US2010/023457
(87) International publication number: WO 2010/101698

(56) References cited:
- WO-A1-03/033581
- WO-A1-2005/082826
- WO-A1-2007/089600
- WO-A2-2009/120457
- WO-A2-2010/078328
- JP-A- 2007 197 654
- US-A1- 2005 209 435
- DATABASE WPI Week 200966 Thomson Scientific, London, GB; AN 2009-N77645 XP002684508, & CN 101 525 416 A (DONGLI FIBRE RES INST CHINA CO LTD) 9 September 2009 (2009-09-09)

## Description

### TECHNICAL FIELD OF THE INVENTION

This invention relates to a method of making bio-based PET packaging and particularly to a method of making and recycling bio-based PET packaging.

### BACKGROUND OF THE INVENTION

As used herein, the term "PET" refers to polyethylene terephthalate, its copolyesters, and combinations thereof in any form including PET flakes, pellets and recycled PET. The term "PET products" refers to products made from PET, including but not limited to resins, performs, and PET packaging. The term "PET packaging" as used herein shall refer to all PET packaging including but not limited to PET packaging used for packaging food products, soft drinks, alcoholic beverages, detergents, cosmetics, pharmaceutical products and edible oils such as PET containers (which encompasses bottles) and PET secondary packaging, which is usually used for organizing and securing for transport, display, and storage of PET containers as well as for advertising the product contained within.

The term "bio-based," as used herein, indicates the inclusion of some component that partially or totally derives from at least one bio-based material. As an example, a "bio-based PET" would be a PET that comprises at least one component that partially or totally derives from at least one bio-based material. The term "bio-based materials" and "renewable materials" both refer to organic materials in which the carbon comes from non-fossil biological sources.

PET is a widely used raw composition for making packaging articles in part due to their excellent combination of clarity, mechanical, and gas barrier properties. Today, most commercial methods produce PET with petrochemically derived raw materials (hereinafter referred to as "petroleum-based PET"). Therefore, the cost of production is closely tied to the price of petroleum. Petroleum-based PET contributes to greenhouse emissions due to its high petroleum derived carbon content. Furthermore, petrochemicals take hundreds of thousands of years to form naturally, making petrochemically-derived products non-renewable, which means they cannot be re-made, re-grown, or regenerated at a rate comparable to its consumption.

As regulations become more rigorous with regard to the environmental impact of industrial activities and as petroleum resources become increasingly scarce, there exists a growing need for a bio-based PET that may serve as an alternative to petroleum-based PET. It would be further desirable if the bio-based PET has similar chemical and/or physical properties and/or chemical structures as petroleum-based PET so that technology and facilities currently designed for petroleum-based PET can be readily applied to bio-based PET. For example, in some applications, it would be desirable if bio-based PET products may be processed through existing petroleum-based PET product manufacturing facilities and/or readily recycled through the systems designed for recycling petroleum-based PET products.

Bio-based materials would also satisfy consumers' increasing demand of products that are environmentally friendly. It would be more desirable if the bio-based materials do not compete with foods or food-grade materials that may potentially increase the costs of necessity items for consumers. For example, the bio-based materials may be obtained from a food or agricultural waste stream. Thus, there is a need to produce PETs derived from bio-based materials that do not compete with foods or food-grade materials.

Other objects, features, and advantages of this invention will be apparent from the following detailed description, drawings, and claims.

WO 2009/120427 discloses a bio-based PET polymer and method of making the same.

### BRIEF DESCRIPTION OF THE FIGURES

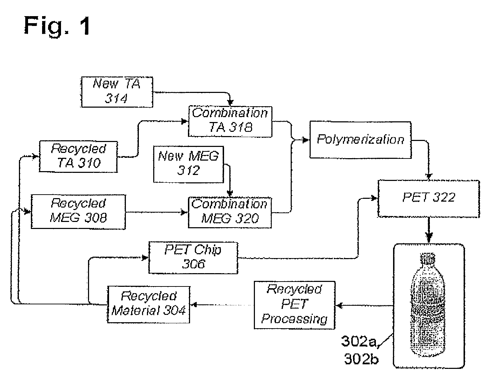

FIG. 1 is a flowchart illustration of a method of recycling a bio-based PET container.

The detailed description explains the embodiments of the invention, together with advantages and features, by way of example with reference to the drawings.

### DETAILED DESCRIPTION OF THE INVENTION

The present invention encompasses forming a bio-based PET from at least one bio-based material. The bio-based PET undergoes solid state polymerization to form a PET resin, which is processed into a PET packaging, a PET preform, and combinations thereof. The bio-based material is selected from forestry waste or agriculture waste. The selection of bio-based materials may depend on factors including, but not limited to, supply availability, cost, type of technology used, and environmental impact.

Steps of method in the embodiments recited herein need not be performed in the order of steps described. One skilled in the art would know which steps may be performed simultaneously or chronologically and at the same or different locations.

The method of the present invention encompasses a method of producing a bio-based PET from at least one bio-based material comprising the following steps:
a) forming at least one PET component from at least one bio-based material, wherein the at least one PET component is selected from a monoethylene glycol ("MEG"), a terephthalic acid ("TA"), and combinations thereof wherein the bio-based material is forestry waste or agricultural waste;
b) processing said bio-based PET component into a bio-based PET;
c) solid state polymerizing the bio-based PET to form a PET resin; and
d) processing the PET resin into a PET product selected from a PET preform, PET packaging, and combinations thereof.

More particularly, the PET component may be a MEG and melt polymerized with a TA to produce a PET. Alternatively, the PET component may be a TA and melt polymerized with a MEG to produce a PET. Yet more alternatively, the PET component may be combinations of MEG and TA, which may be melt polymerized to produce a PET. More particularly, the melt polymerization step further comprises mixing the MEG and the TA in a catalyst solution, promoting esterification between the MEG and TA at atmospheric pressure to form a bio-based PET, optionally separating the impurities from the bio-based PET, and polycondensing the bio-based PET.

More particularly, the MEG and the TA may be produced from the bio-based material using methods including but not limited to fast pyrolysis, acid hydrolysis, enzymatic hydrolysis, microbial degradation, mycological degradation, and hydrogenolysis.

Alternatively, the bio-based material is used to produce a PET packaging, wherein the PET packaging comprises an edible product. In another embodiment, the bio-based material may be used to produce an ingredient and the edible product further comprises the ingredient. In yet another embodiment, the ingredient may be selected from sugar, ethanol, carbon dioxide, and combinations thereof.

The PET resin produced using the method of the invention may be molded into a PET container by various methods including but not limited to making preforms, blowing vessels, thermo forming, extrusion molding, compression molding, injection molding, extrusion blow molding and other methods. A skilled artisan would be able to determine which method is more suitable for each application considering factors including but not limited to time, cost, availability, location, design of the vessel, and function of the vessel. The PET container may be used but is not limited to packaging food products, soft drinks, alcoholic beverages, detergents, cosmetics, pharmaceutical products, edible oils, and combinations thereof.

### Method of Producing Bio-Based PET: Sugarcane

Today, a typical shortcoming of sugarcane refining is that after the sugarcane has been refined into sugar and molasses, the leftover cane husks (or sometimes called bagasse) are often discarded in landfills or burned for fuel or used for animal feed. Bagasse is rich in cellulose, hemicelluloses, and lignin but has practically no food value. Finding alternative ways to use the leftover bagasse to produce a bio-based PET would reduce waste.

### Method of Producing Bio-Based PET: Agricultural Waste Streams

A particular embodiment encompasses a method of producing a bio-based PET from agricultural waste comprising the following steps: a) collecting an agricultural waste stream; b) refining the agricultural waste stream into a MEG; and c) melt polymerizing the MEG with a TA to form a PET. In a more particular embodiment, the TA may be a bio-based TA. The method further comprises solid state polymerizing the PET to form a PET resin and processing the PET resin into a PET product selected from a PET preform, PET packaging, and combinations thereof. More particularly, the agricultural waste stream may be selected from sugar husk, bagasse, corn stover, woodchips, other agricultural waste streams and products, and combinations thereof.

### Recycling bio-based PET Packaging

Once a bio-based PET packaging is filled with a product, the bio-based PET packaging may be distributed to a consumer through marketing outlets and other means. After the product is removed or consumed, the used bio-based PET packaging may be collected in a recycle supply chain. The recycle supply chain may include, but is not limited, one or more of the an organized array of curb side pickup, special containers available to the public in building, at events, and in other locations, using designated collection sites, and municipal recycling programs. After entering the recycle supply chain, the used bio-based PET packaging may be processed into a PET chip. The term "PET chip" as used herein refers to PET resin in the forms of chips (or sometimes referred to as pellets) and flakes that are primarily made form used PET packaging, including used bio-based PET packaging and used petroleum-based PET packaging. PET chips typically require only minimal cleaning and re-melting in order to be used in a new PET packaging.

The used bio-based PET packaging may also be processed to a recycled MEG or a recycled TA by chemical depolymerization methods such as hydrolysis, methanolysis, glycolysis, alcoholysis, aminolysis and combinations thereof. The PET chip, the recycled MEG, and/or the recycled TA may be further processed to form new bio-based PET products. Under the industrial recycling operations available today, existing recycle supply chains are unlikely to recover a sufficient amount of used bio-based PET packaging to generate all the new PET products in demand. Thus, a supply of new bio-based MEG and new bio-based-TA will need to be continually produced to satisfy demands.

Fig. 1 shows a method of recycling a used bio-based PET packaging 302a comprising the steps of: a) processing a used bio-based PET 302a through a PET processing center to produce at least one recycled material 304 selected from a PET chip 306, a recycled MEG 308, a recycled TA 310, and combinations thereof. Alternatively, the method further comprises separating the at least one recycled material 304 into groups of PET chips 306, recycled MEG 308, and recycled TA 310. In one embodiment, the recycled material is a PET chip 306 and the method further comprises routing the PET chip to a molding process. Alternatively, the recycled material is a recycled MEG 308 or a recycled TA 310 and the method optionally comprises combining the recycled MEG 308 with a new bio- based MEG 312 to form a combination MEG and combining the recycled TA 310 with a new bio-based TA 314 to form a combination TA. More particularly, the method comprises polymerizing the combination MEG and the combination TA to form a new PET. Yet more particularly, the method comprises combining the recycled MEG with the new MEG at a specific ratio. Even more particularly, the method comprises combining the recycled TA with the new TA at a specific ratio. Alternatively, the new PET may be combined with the PET chip 306 to produce a PET container 302b.

Thus, a sustainable bio-based packaging may be created by utilizing both new MEG and TA made from fresh bio-based materials and recycled MEG and TA made from recycled bio-based materials.

The capabilities of the present invention can be implemented in software, firmware, hardware or combinations thereof.

As one example, one or more aspects of the present invention can be included in an article of manufacture (e.g., one or more computer program products) having, for instance, computer usable media. The media has embodied therein, for instance, computer readable program code means for providing and facilitating the capabilities of the present invention. The article of manufacture can be included as a part of a computer system or sold separately.

Additionally, at least one program storage device readable by a machine, tangibly embodying at least one program of instructions executable by the machine to perform the capabilities of the present invention can be provided.

While the preferred embodiment to the invention has been described, it will be understood that those skilled in the art, both now and in the future, may make various improvements and enhancements which fall within the scope of the claims which follow.

## Claims

1. A method of making a bio-based polyethylene terephthalate (PET) product, comprising:
(a) forming at least one PET component from at least one bio-based material, wherein the at least one PET component is selected from a monoethylene glycol ("MEG"), a terephthalic acid ("TA"), and combinations thereof and wherein the bio-based material is forestry waste or agricultural waste;
(b) processing said bio-based PET component into a bio-based PET;
(c) solid state polymerizing the bio-based PET to form a PET resin; and
(d) processing the PET resin into a PET product selected from a PET preform, PET packaging, and combinations thereof.

2. The method of claim 1, wherein said PET product is a container or secondary PET packaging.

3. The method of claim 2, wherein the PET product is a container.

4. The method of any of claims 1 to 3, wherein the at least one PET component is produced from the bio-based material using methods of fast pyrolysis, acid hydrolysis, enzymatic hydrolysis, microbial degradation, mycological degradation, or hydrogenolysis.

5. The method of claim 1, wherein said at least one PET component is MEG.

6. The method of claim 1, wherein said at least one PET component is MEG and the processing step (b) comprises melt polymerising the MEG with TA to form a bio-based PET.

7. The method of claim 1, wherein the PET product is a bottle.

8. The method of claim 3, where the PET resin is molded into a container by a method selected from making preforms, blowing vessels, thermo forming, extrusion molding, compression molding, injection molding, and extrusion blow molding.

## Patentansprüche

1. Verfahren zum Herstellen eines Polyethylenterephthalat(PET)-Produkts auf biologischer Basis, umfassend:
(a) Bilden von wenigstens einer PET-Komponente aus wenigstens einem Material auf biologischer Basis, wobei die wenigstens eine PET-Komponente ausgewählt ist aus einem Monoethylenglycol ("MEG"), einer Terephthalsäure ("TA") und Kombinationen davon und wobei das Material auf biologischer Basis forstwirtschaftlicher Abfall oder landwirtschaftlicher Abfall ist;
(b) Verarbeiten der PET-Komponente auf biologischer Basis zu einer PET auf biologischer Basis;
(c) Feststoffpolymerisieren der PET auf biologischer Basis, um ein PET-Harz zu bilden; und
(d) Verarbeiten des PET-Harzes zu einem PET-Produkt ausgewählt aus einer PET-Vorform, einer PET-Verpackung und Kombinationen davon.

2. Verfahren gemäß Anspruch 1, wobei das PET-Produkt ein Behälter oder eine PET-Sekundärverpackung ist.

3. Verfahren gemäß Anspruch 2, wobei das PET-Produkt ein Behälter ist.

4. Verfahren gemäß einem der Ansprüche 1 bis 3, wobei die wenigstens eine PET-Komponente unter Verwendung von Verfahren der schnellen Pyrolyse, der Säurehydrolyse, der enzymatischen Hydrolyse, des mikrobiellen Abbaus, des mykologischen Abbaus oder der Hydrogenolyse aus dem Material auf biologischer Basis hergestellt wird.

5. Verfahren gemäß Anspruch 1, wobei die wenigstens eine PET-Komponente MEG ist.

6. Verfahren gemäß Anspruch 1, wobei die wenigstens eine PET-Komponente MEG ist und der Verfahrensschritt (b) Schmelzpolymerisation des MEG mit TA zum Bilden eines PET auf biologischer Basis umfasst.

7. Verfahren gemäß Anspruch 1, wobei das PET-Produkt eine Flasche ist.

8. Verfahren gemäß Anspruch 3, wobei das PET-Harz durch ein Verfahren ausgewählt aus der Herstellung von Vorformen, Blasen von Gefäßen, Thermoformen, Strangpressen, Formpressen, Spritzgießen und Extrusionsblasen zu einem Behälter geformt wird.

## Revendications

1. Procédé de fabrication d'un produit en poly(téréphtalate d'éthylène) (PET) d'origine biologique, comprenant :
(a) la formation d'au moins un constituant de PET à partir d'au moins un matériau d'origine biologique, l'au moins un constituant de PET étant choisi parmi un monoéthylèneglycol (« MEG »), un acide téréphtalique (« TA ») et des associations de ceux-ci et le matériau d'origine biologique étant un déchet forestier ou un déchet agricole ;
(b) la transformation dudit constituant de PET d'origine biologique en un PET d'origine biologique ;
(c) la polymérisation à l'état solide du PET d'origine biologique pour former une résine de PET ; et
(d) la transformation de la résine de PET en un produit en PET choisi parmi une préforme en PET, un emballage en PET et des associations de ceux-ci.

2. Procédé selon la revendication 1, dans lequel ledit produit en PET est un récipient ou un suremballage en PET.

3. Procédé selon la revendication 2, dans lequel le produit en PET est un récipient.

4. Procédé selon l'une quelconque des revendications 1 à 3, dans lequel l'au moins un constituant de PET est produit à partir du matériau d'origine biologique à l'aide de procédés de pyrolyse rapide, d'hydrolyse acide, d'hydrolyse enzymatique, de dégradation microbienne, de dégradation fongique ou d'hydrogénolyse.

5. Procédé selon la revendication 1, dans lequel ledit au moins un constituant de PET est le MEG.

6. Procédé selon la revendication 1, dans lequel ledit au moins un constituant de PET est le MEG et l'étape de transformation (b) comprend la polymérisation à l'état fondu du MEG avec du TA pour former un PET d'origine biologique.

7. Procédé selon la revendication 1, dans lequel le produit en PET est une bouteille.

8. Procédé selon la revendication 3, dans lequel la résine de PET est moulée en un récipient par un procédé choisi parmi la fabrication de préformes, le soufflage de récipients, le thermoformage, le moulage par extrusion, le moulage par compression, le moulage par injection et le moulage par extrusion et soufflage.
